# EUROPEAN PATENT APPLICATION

(11) **EP 1 726 208 A1**
(43) Date of publication of application: **29.11.2006**
(21) Application number: 05076219.4
(22) Date of filing: 24.05.2005
(51) Int. Cl.: A01K 67/027, C12N 5/06, C12N 15/12

(54) **Murine stem cells and applications thereof**

(71) Applicant: Centro de Investigacion Biomolecular Aplicada S.L., 37004 Salamanca (ES)
(72) Inventor: Sanchez-Garcia, Isidro, 37004 Salamanca (ES); Voces Sanchez, Felipe, 37004 Salamanca (ES)
(74) Representative: ABG Patentes, S.L.

(57) **Abstract**

The invention relates to murine stem cells capable of specifically expressing in stem cells human genetic anomalies associated with human pathologies. Applications of said stem cells, such as human diseases diagnostic, therapeutic and prophylactic applications, products and methods are provided.

## Description

### FIELD OF THE INVENTION

The invention relates to murine stem cells capable of specifically expressing in stem cells human genetic anomalies associated with human pathologies. Applications of said stem cells, such as human diseases diagnostic, therapeutic and prophylactic applications, products and methods are provided.

### BACKGROUND OF THE INVENTION

Stem cells (SC) are defined as cells that have the ability to perpetuate themselves through self-renewal and to generate mature cells of a particular tissue through differentiation. Examples of SCs include haematopoietic stem cells, neural stem cells, cancer stem cells, etc.

Nowadays, it is known that cancers consist of heterogeneous populations of cancer cells that differ markedly in their ability to proliferate and form new tumours. While the majority of the cancer cells have a limited ability to divide, a population of cancer stem cells (CSC) which has the exclusive ability to extensively proliferate and form new tumours can be identified based on marker expression.

Growing evidence suggests that pathways regulating the self-renewal of normal stem cells are deregulated in CSCs resulting in the continuous expansion of self-renewing cancer cells and tumour formation. This suggests that agents targeting the defective self-renewal pathways in cancer cells might lead to improved outcomes in the treatment of these diseases.

This strategy has implications for the biology of tumour formation as well as the diagnosis and treatment of cancer. To treat cancer effectively, the CSCs must be eliminated. Otherwise, the tumour will rapidly reform if the therapy eliminates non-tumourigenic cancer cells but spares a significant population of the CSCs. Classically, treatments for cancer have relied on the ability to shrink tumours. Since in many cases the CSCs represent a minority cell population of the tumour, agents selectively killing the CSCs are likely overlooked in our current screening methods, which rely on rapid reduction of tumour size.

If an agent spares a significant number of the CSCs, then the remaining cells could rapidly reform the tumour. In addition to its effect on our understanding of the efficacy of our current therapies, the stem cell model for cancer is likely to affect the identification of future therapeutic targets. By directing expression analyses to enriched population of tumorigenic cancer cells, the identification of novel diagnostic markers and novel therapeutic targets should be more effective. In addition, it is becoming apparent that treatments that directly target the pathways involved in maintenance of CSCs would have a significantly greater chance of success.

If it is assumed that the genetic alteration responsible for the cancer development takes place in the CSCs, mouse models based on this issue can be designed. New strategies must focus on determining what is the process responsible for the maintenance of the CSCs phenotype. This will be the aim for the new identification of targets to design drugs against them. Therefore, mouse models generated this way will be the basic tool to design drugs in this context, against the maintenance of the CSCs.

Many of the new anticancer agents target unconventional aspects of cancer development and interact with other drugs in an unpredictable manner. They are predicted to show clinical benefit in only small subpopulations of patients

In addition, in order to improve the efficacy of current therapies, the stem cell model for cancer will also help for the identification of potential therapeutic targets. By directing expression analyses to enriched population of tumourigenic cancer cells, the identification of novel diagnostic markers and novel therapeutic targets will be more effective. Recent evidence also indicates that treatments directly targeting the pathways involved in maintenance of CSCs will have significantly greater chances of success.

Based on the understanding that the genetic alteration responsible for cancer development takes place in the CSCs, it is possible to design mouse models that accurately reproduce this genetic alteration. First, we must determine what is responsible for the maintenance of the CSC phenotype since this will be the basis for the identification of targets and the potential drugs to be used against them. Therefore, mouse models generated following this strategy will represent the basic tool in designing drugs to inhibit CSC maintenance.

### BRIEF DESCRIPTION OF THE DRAWINGS

Figure 1 shows transgene, thymidine kisase and BCR-ABLp210, expression in Scal⁺Lin⁻ and Scal⁻Lin⁺ lines.
Figure 2 shows the percentage of mice developing tumors after ganciclovir treatment and saline solution treatment (control).

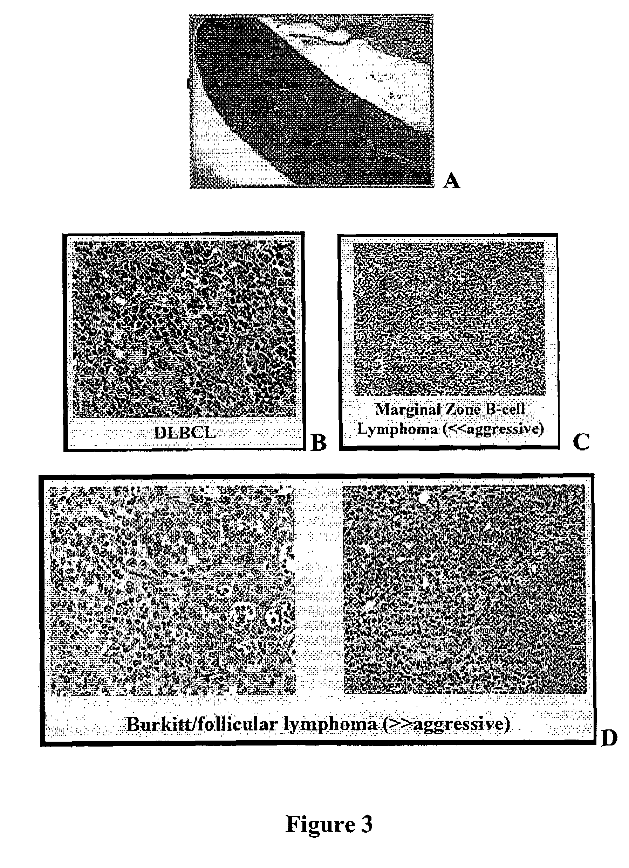

In Figure 3 three representative pictures of (a) normal spleen, (b) a spleen which has developed DLBCL, (c) a spleen which has developed a Burkitt lymphoma less aggressive, and (d) a spleen which has developed a Burkitt lymphoma more aggressive are shown.
Figure 4 is a graphical description of expression data for the known target genes in the bone marrow cancer stem cell of the Sca1+ Bcl6 mice. Each gene (identified at right) represented by a single row of colored boxes; each mouse is represented by a single column.
Figure 5 is a graphical description of the expression pattern in the Bcl6 mouse model of some of the described genes up/down regulated in the human Diffuse Large B-cell Lymphoma (DLBCL).

### DETAILED DESCRIPTION OF THE INVENTION

To facilitate the understanding of the instant description, the meaning of some terms and expressions in the context of the invention will be explained below.

The term "stem cell" (SC) refers to a cell that has the ability to perpetuate itself through self-renewal and to generate mature cells of a particular tissue through differentiation. Examples of SCs include haematopoietic stem cells, neural stem cells, etc.

The term "cancer stem cell" (CSC) refers to a cell that has the ability to extensively proliferate and form new tumours, i.e., cells with indefinite proliferative potential that drive the formation and growth of tumours; said term includes both gene alteration in SCs and gene alteration in a cell which becomes a CSC. In a preferred embodiment, said CSCs are Sca1⁺.

The term "subject" refers to members of mammal species, and includes, but is not limited to, domestic animals, roedent, primates and humans; the subject is preferably a human being, male or female, of any age or race.

The term "murine" includes mice, rats, guinea pigs, hamsters and the like; in a preferred embodiment the murine animal is a mouse.

The expression "gene that is created and/or activated by a genetic anomaly associated with a human pathology", hereinafter, the "activatable gene", refers to a gene, altered gene (including mutations, deletions, insertions, duplications, etc.) or gene fusion that, when incorporated into the genome of a mammal, reproduces the human pathology with which said gene, altered or gene fusion is associated. The term "human pathology" includes human pathology of stem cell origin as well as human pathology of non stem cell origin. In a particular embodiment, the human pathology is a human pathology of stem cell origin. In another particular embodiment, said activatable gene is a gene that is created and/or activated by a genetic anomaly associated with a human pathology of stem cell origin such as a human tumoral pathology or a human non-tumoral pathology; in a further particular embodiment said human pathology is selected from epithelial or mesenchimal cancer, for example, human lymphomas, leukaemias, sarcomas and carcinomas, such as, chronic myeloid leukaemia, B-cell acute lymphoblastic leukaemia, T-cell acute lymphoblastic leukaemia, acute myeloid leukacmia, chronic myeloid leukaemia, lymphoproliferative syndromes, multiple myeloma, liposarcoma, Ewing sarcoma, lung carcinoma, breast carcinoma, skin carcinoma and kidney carcinoma. In another particular embodiment, said activatable gene is a gene that is created and/or activated by a genetic anomaly associated with a human pathology such as haematopoietic or embryonic stem cell migration. In another particular embodiment, said activatable gene is a gene that is created and/or activated by a genetic anomaly associated with a human pathology such as neurological disorders, rare diseases, metabolic diseases, immunological diseases, cardiovascular diseases, etc.

Illustrative, non limitative examples of said activatable gene include the genes identified as BCR-ABL^{p210}, BCR-ABL^{p190}, Slug, Snail, HOX11, RHOM2/LMO-2, TAL1, Maf-B, FGFR, c-maf, MMSET, BCL6, BCL10, MALT1, cyclin D1, cyclin D3, SCL, LMO1, LMO2, TEL-AML1, E2A-HLF, E2A-Pbx1, TEL-ABL, AML1-ETO, FUS-CHOP, FUS-DDIT3, EWS-WT1, EWS FLI1, EWSR1-DDIT3, FUS-ATF1, FUS-BBF2H7, etc. Information concerning BCR-ABL^{p210}, BCR-ABL^{p190}, Slug, Snail, HOX11, RHOM2/LMO-2 and TAL1 genes can be found on WO 03/046181. Maf-B, FGFR, c-maf, and MMSET genes are related to multiple myeloma; BCL6, BCL10, MALT1, cyclin D1 and cyclin D3 are related to lymphoproliferative disorders such as Burkitt-like lymphoma, Diffuse Large B-Cell Lymphoma (DLBCL) or marginal zone lymphoma; SCL, HOX11, LMO1 and LMO2 are related to T-cell acute lymphoblastic leukaemia; BCR-ABL^{p190}, TEL-AML1, E2A-HLF and E2A-Pbx1 are related to B-cell acute lymphoblastic leukaemia; TEL-ABL, AML1-ETO and FUS-ERG are related to acute myeloid leukaemia; BCR-ABL^{p210} is related to chronic myeloid leukaemia; FUS-CHOP is related to liposarcoma; EWS-WT1 and EWS FLI1 are related to Ewing sarcoma; FUS-DDIT3 and EWSR1-DDIT3 are related to myxoid liposarcoma; FUS-ATF1 is related to angiomatoid fibrous histiocytoma; and FUS-BBF2H7 is related to low grade fibromyxoid sarcoma (LGFMS). The sequences of the above mentioned activatable genes arc known from the prior art.

The term "sample", as used in the instant invention, can be any biological sample susceptible of containing SCs or CSCs, such as a liquid sample, for example, blood, serum, etc., or a solid sample, such a tissue sample. The sample can be obtained by any conventional method, including surgical resection in case of solid samples. The sample can be obtained from subjects previously diagnosed, or not diagnosed, with a human pathology of stem cell origin; or also from a subject undergoing treatment, or who has been previously treated for a said human pathology of stem cell origin.

In an aspect, the invention relates to a murine stem cell (mSC) comprising a gene created and/or activated by a genetic anomaly associated with a human pathology. In a particular embodiment, said mSC is a murine cancer stem cell (mCSC); preferably, said mCSC is Scal⁺. In a particular embodiment, said human pathology is a human pathology of stem cell origin. Examples of human pathologies have been mentioned previously.

In an embodiment, the mSC or the mCSC provided by the invention further comprises a marker, such as a marker useful for the specific isolation and/or identification of cancer stem cells, or for the procurement of cancer stem cells, or for the differentiation of cancer stem cells from health stem cells. Virtually any product from a SC or from a CSC capable of achieving said aims can be used, for example, genes, proteins, etc. Information concerning said markers can be used for designing assays for isolating and/or identifying SCs and/or CSCs, or for the procurement of SCs and/or CSCs, or for the differentiation of CSCs from health SCs. Due to the similarity between the mouse genome and the human genome, markers useful for the above mentioned aims may be also used for achieving the same aims in human SCs (hSCs) and/or human CSCs (h(CSCs).

In a further embodiment, the mSC or the mCSC provided by the invention further comprises a reporter. A reporter, according to the instant invention, is a product which is attached to the surface of the cell membrane, or introduced inside the SC or CSC, which allows for spatiotemporal identification of thc onset, progression, dissemination and further physiopathological processes as well as the effect of therapies by molecular imaging techniques. Illustrative, non-limitative molecular imaging techniques which can be applied include positron emission tomography (PET), computed tomography (TAC), nuclear magnetic resonance (NMR), high performance X-ray, etc., methods based on the emission of bioluminiscent signals based on enzymatic reactions, methods based on the insertion of genetic constructions containing a label, such as green fluorescent protein (GFP), etc. Virtually any reporter capable of achieving said aims can be used. Said reporter can also play a role in diagnostic assays (e.g., molecular fingerprinting of a human disease, etc.), drug discovery and development processes, target identification as well as in improving the efficacy and reliability of all phases of the clinical development.

The mSCs and/or mCSCs provided by the instant invention can be used as a biomarker for in a number of strategies, such as, for example, for:
- detecting the presence of a gene created and/or activated by a genetic anomaly associated with a human pathology in a subject, or for
- assessing the risk or predisposition of a subject to develop a human pathology in a subject, or for
- determining the stage or severity of a human pathology in a subject, or for
- monitoring the effect of the therapy administered to a subject having a human pathology in a subject, or for
- designing an individualized therapy for a subject suffering from a human pathology in a subject, or for
- therapeutic monitoring and evaluation of therapeutic benefits; or for
- designing human clinical trials, or for
- diagnosis of cancer and/or specific processes and effects of cancer development, like cancer dissemination; or for
- patient selection for personalized therapeutics; or for
- drug discovery and pharmacokinetics guidance.

The use of the mSCs and/or mCSCs provided by the instant invention as a biomarker for the above mentioned strategies constitutes an additional aspect of the instant invention.

Moreover, the mSCs and/or mCSCs provided by the instant invention can also be used for discovering, screening, scarching, identifying, evaluating and validating targets for human pathologies, or for identifying specific genes related to self-renewal ability of cancer stem cells. Advantegcously, said genes related to self-renewal ability are specific only of cancer stem cells (not of health stem cells). The use of the mSCs and/or mCSCs provided by the instant invention for the previously above mentioned purposes constitutes an additional aspect of the instant invention.

In another aspect, the invention relates to a method for detecting the presence of a gene created and/or activated by a genetic anomaly associated with a human pathology in a subject or for assessing the risk or predisposition of a subject to develop said pathology which comprises identifying a SC in a sample from said subject, said SC comprising a genetic anomaly associated with said human pathology. In a particular embodiment, said SC is a CSC. The above method can be performed *in vitro* or *in vivo*. Accordingly, the identification of said SCs or CSCs in a sample from the subject can be both diagnostic or prognostic of human pathologies in said subject. For example, the identification of said SCs or CSCs in a sample from the subject is indicative of human pathologies or a greater risk or predisposition of the subject to develop any of said pathologies.

Said SCs or said CSCs can be identified by conventional techniques in view of the markers and/or reporters to be identified. In an embodiment, said cells can be identified by means of antibody-antigen cross-reactions, by means of ligand-rcccptor interactions, by molecular imaging techniques, etc. The above method can be performed *in vitro* or *in vivo.*

There is a wide variety of immunological assays available for detecting and quantifying the formation of specific antigen-antibody complexes; numerous competitive and non-competitive protein binding assays have previously been disclosed, and a large number of these assays are commercially available.

Thus, when the marker or reporter to be detected acts as an antigen, said product can be quantified with antibodies such as, for example, monoclonal antibodies, polyclonal antibodies, intact or recombinant fragments thereof, "combibodies" and Fab or scFv antibody fragments, specific against said products; these antibodies being human, humanized or of non-human origin. The antibodies used in these assays can be labeled or not; the unlabeled antibodies can be used in agglutination assays; the labeled antibodies can be used in a wide variety of assays. Marker molecules which can be used to label antibodies include radionucleotides, enzymes, fluorophores, chemoluminescent reagents, enzyme substrates or cofactors, enzyme inhibitors, particles, colorants and derivatives.

There is a wide range of well known assays which can be used in the present invention using unlabeled antibodies (primary antibody) and labeled antibodies (secondary antibody); included among these techniques are the Western-blot or Western transfer, ELISA (Enzyme-Linked Immunosorbent Assay), RIA (Radioimmunoassay), competitive EIA (Competitive Enzyme Immunoassay), DAS-ELISA (Double Antibody Sandwich-ELISA), immunocytochemical or immunohistochemical techniques, techniques based on the use of biochips or microarrays of proteins including specific antibodies, or assays based on colloidal precipitation in formats such as dipsticks. Other ways to detect and quantify said products (markers or reporters) include affinity chromatography techniques, ligand binding assays and the like.

In another aspect, the invention refers to a method for determining the stage or severity of a human pathology in a subject or monitoring the effect of the therapy administered to a subject having said pathology, which comprises identifying and quantifying the concentration of SCs in a sample from said subject, said SCs comprising a gene created and/or activated by a genetic anomaly associated with a human pathology, and comparing said concentration with that of a control sample or with a prior sample from said subject or with a prior sample from said subject taken before administering a therapy. In a particular embodiment, said SC is a CSC. Said SCs or said CSCs can be identified and quantified as previously mentioned by any suitable conventional technique. The above method can be performed *in vitro* or *in vivo.* This method further comprises the step of comparing the concentration of SCs in a sample from said subject, said SCs comprising a gene created and/or activated by a genetic anomaly associated with a human pathology, with that of a control sample or with a prior sample from said subject or with a prior sample from said subject taken before administering a therapy. The control sample can be obtained from subjects free of human pathologies. According to this method, it is possible to determine the stage or severity of a human pathology in a subject or to monitor the effect of the therapy administered to a subject having said pathology.

In another aspect, the invention relates to a method for discovering, screening, searching, identifying, developing and/or evaluating compounds for treating a human pathology, or for repositioning known drugs or combinations of compounds, which comprises contacting a candidate compound with a mSC or a mCSC provided by the instant invention, and monitoring the response. A compound which abolishes or slows down the proliferation of said cells or a compound which kills said cells can be selected for further assays. This method can be performed both *in vitro* or *in vivo*, for example, by xenografting SCs or CSCs into immunodeficient mice, etc.

It is important to mention that the development of molecular and pharmacological therapeutics to successfully treat and prevent human pathologies, for example, pathologies of stem cell origin, such as cancer, will allow a precise assessment of the therapeutical potential of any strategy before the application in human therapy.

In another aspect, the invention provides a compound which abolishes or slows down the proliferation of SCs or CSCs, e.g., cells of murine origin, or a compound which kills said cells. Said compound can be identified and evaluated according to the above method and is potentially useful for treating a human pathology. In an embodiment, said compound is selected from the formed by:
a) an antibody, or combination of antibodies, specific against one or more epitopes present in said SCs or CSCs, e.g., mSCs or mCSCs; and
b) cytotoxic agents, such as antibiotics, toxins, compounds with radioactive atoms, or chemotherapeutic agents, which include, without limitation, small organic and inorganic molecules, peptides, phosphopeptides, antisense molecules, ribozymes, siRNAs, triple helix molecules, etc., which abolish or slow down the proliferation of said SCs or CSCs, e.g., mSCs or mCSCs, or kill said cells.

Another aspect of the invention relates to the use of the above mentioned compound which abolishes or slows down the proliferation of SCs or CSCs, e.g., mSCs or mCSCs, or kills said cells, in the manufacturing of a pharmaceutical composition for the treatment of a human pathology.

Thus, in another aspect, the invention relates to a pharmaceutical composition comprising a therapeutically effective amount of one or more compounds of those previously mentioned which abolish or slow down the proliferation of SCs or CSCs, e.g., mSCs or mCSCs, or kill said cells, together with one or more cxcipicnts and/or carrier substances. Said pharmaceutical composition may also contain any other active ingredient useful for treating a human pathology. In a particular embodiment, said human pathology is a human pathology of stem cell origin.

The excipients, carrier substances and auxiliary substances must be pharmaceutically and pharmacologically tolerable, such that they can be combined with other components of the formulation or preparation and do not exercise adverse effects on the treated organism. Pharmaceutical compositions or formulations include those which are suitable for oral or parenteral administration (including subcutaneous, intradermal, intramuscular and intravenous), although the best administration route depends on the subject's condition. The formulations can be in single dose form. The formulations are prepared according to methods known in the field of pharmacology. The amounts of active substances to be administered can vary according to the particularities of therapy.

Another object of the invention consists in a kit for carrying out the present invention. Thus, an embodiment of the present invention provides a kit that comprises an antibody specific against a marker or a reporter eventually present in SCs or CSCs and a carrier in suitable packing, wherein said antibody is, for example, a monoclonal antibody, a polyclonal antibody, an intact or recombinant fragment thereof, a "combibody" or a Fab or scFv antibody fragment; said antibody being human, humanized or of non-human origin. Said antibody can be labeled or not; the unlabeled antibody can be used in agglutination assays; the labeled antibody can be used in a wide variety of assays. Marker molecules which can bc used to label antibodies include radionucleotides, enzymes, fluorophores, chemoluminescent reagents, enzyme substrates or cofactors, enzyme inhibitors, particles, colorants and derivatives. The kit can also contain a combination of said antibodies.

The kits of the invention can be employed, among other uses, for detecting the presence of SCs or CSCs, said cells containing a gene created and/or activated by a genetic anomaly associated with a human pathology of stem cell origin, or for assessing the risk or predisposition of a subject to develop a human pathology of stem cell origin in a subject, or for determining the stage or severity of a human pathology of stem cell origin in a subject, or for monitoring the effect of the therapy administered to a subject having a human pathology of stem cell origin (e.g., for therapeutic monitoring and evaluation of therapeutic benefits), or for designing an individualized therapy for a subject suffering from a human pathology of stem cell origin, or for designing human clinical trials, or for the diagnosis of cancer and/or specific processes and effects of cancer development, like cancer dissemination; or for patient selection for personalized therapeutics; or for drug discovery and pharmacokinetics guidance; or for discovering, screening, searching, identifying, evaluating and validating targets for human pathologies, or for identifying specific genes related to self-renewal ability of cancer stem cells.

In another aspect the invention refers to a method for designing an individualized therapy for a human suffering from a human pathology which comprises selecting a compound identified as previously mentioned, wherein said compound abolishes or slows down the proliferation of SCs or CSCs or kills said cells, said compound capable of being used as active principle in a pharmaceutical composition to be administered to said subject. This method can be performed both *in vitro* or *in vivo*.

In another aspect, the invention relates to a method for designing human clinical trials which comprises:
a) selecting targets for mSCs or mCSCs provided by the instant invention or molecular profiling said cells;
b) validating said target or said molecular profile in a disease state; and
c) selecting responders vs non-responders for a particular treatment.

In another aspect, the invention provides a DNA construct, hereinafter the DNA construct of the invention, that comprises an activatable gene (i.e., a gene created and/or activated by a genetic anomaly associated with a human pathology) operatively bound to a promoter that directs the expression of said genetic anomaly in Sca-1⁺ cells, wherein said genetic anomaly is selected from (i) a nucleic acid comprising a BCL6 gene and (ii) a nucleic acid comprising a first nucleotide sequence coding for a kinase and a second nucleotide sequence comprising BCR-ABL^{p210}.

In a particular embodiment, the DNA construct of the invention comprises a nucleic acid comprising a first nucleotide sequence coding for hcrpcx simple thymidine kinase (HSV-tk), a second nucleotide sequence comprising BCR-ABL^{p210}, and a third nucleotide sequence comprising an internal ribosome-entry site (IRES) sequence, wherein 3' end of said first nucleotide sequence is bound to the 5' end of said third nucleotide sequence, and the 3' end of said third nucleotide sequence is bound to the 5' end of said second nucleotide sequence.

The promoter that directs expression of the activatable gene in Sca-1⁺ cells is a sequence of nucleic acid implicated and necessary in the initiation of transcription, which directs the expression of the activatable gene in said cells, and includes the binding site of RNA polymerase. Within the context of the present invention, the term "promoter" may include other sites to which the transcription regulating proteins can bind. In a particular embodiment, the promoter that directs the expression of the activatable genes in Sca-1⁺ cells is the pLy-6E1 promoter of mouse or a functional fragment thereof, in other words, it is able to direct the tissue specific expression of the different transgenes in mice. The pLy-6E1 promoter is well characterised and contains all the elements necessary for the selective expression in Sca-1<+> cells [Miles C., Sanchez M-J, Sinclair A, and Dzierzak, E. (1997) "Expression of the Ly-6E.1 (Sca-1) transgene in adult haematopoietic stem cells and the developing mouse embryo". Development 124:537-547]. Thus, in a particular embodiment, the promoter that directs the expression of said activatable gene in Sca-1⁺ cells is the mouse promoter pLy-6E.1 or a functional fragment thereof.

The expression "operatively bound" relates to the orientation of the promoter with respect to sequence of activatable gene. The promoter is placed such that it is able to control or regulate the expression of said activatable gene.

The DNA construct of the invention can be easily obtained by conventional digestion methods with restriction and binding enzymes, and similar enzymes as described by Sambrook, Fitsch and Maniatis, eds., (1989) "Molecular Cloning: A Laboratory Manual". Cold Spring Harbor Laboratory Press, Cold Spring Harbor NY.

The DNA construct of the invention can be used, if desired, for the production of vectors useful for transforming mammal embryos and for generating transgenic animals using conventional methods such as those described by Sambrook et al., cited above.

Alternatively, the DNA construct of the invention can be used for obtaining a linear fragment of DNA useful for microinjection of DNA into fertilised oocytes in order to generate transgenic animals. Said linear fragment of DNA useful for microinjection can be obtained by means of cutting with restriction enzymes in order to obtain a linear DNA fragment that comprises the activatable gene.

In another aspect, the invention provides a transgenic non-human mammal that contains on its genome a DNA construct of the invention. The transgenic non-human mammal provided by this invention possesses, as a result, a genotype that confers a greater tendency to develop human pathology (e.g., of stem cell origin or, alternatively, of non stem cell origin) when compared to the non-transgenic mammal. Said non-human mammal is useful for studying said pathology among other goals and for evaluating potentially useful compounds for treating and/or preventing said pathology.

The expression "non-human mammal", as is used in this description, includes any non-human animal belonging to the class of mammals, for example, mice.

In a particular embodiment, the non-human transgenic animal provided by the invention are those described in the Examples accompanying the description.

For the generation of the transgenic non-human mammal provided by this invention, the DNA construct of the invention has been introduced into said mammal, or into a predecessor thereof, in an embryonic state, for example, in the state of a cell, or fertilized oocyte and, generally, not later than the g cell state.

Therefore, the invention provides a procedure for the preparation of a transgenic non-human mammal that possesses a genetic anomaly associated with a human pathology of stem cell origin, that comprises
(i) introducing a DNA construct of the invention into a fertilized oocyte of a non-human transgenic mammal;
(ii) implanting said fertilized oocyte into a pseudopregnant wet nursing mother to produce descendants; and
(iii) analysing said descendants to evaluate the existence of activated genes and/or genes created by a genetic anomaly associated with a human pathology of stem cell origin.

In a particular embodiment, said genetic anomaly associated with a human pathology is a human pathology of stem cell origin, in which case, the descendents are analysed to evaluate the existence of activated genes and/or genes created by the genetic anomaly associated with the human pathology of stem cell origin in question.

There are different means conceived in the state of the art by which a sequence of nucleic acid can be introduced into an embryo of an animal such that it can be incorporated geneticly in an active state, all of which can be applied to the generation of transgenic non-human mammals of the present invention. A method consists of transfecting the embryo with said sequence of nucleic acid as occurs naturally, and selecting the transgenic animals in which said sequence has been integrated onto the chromosome at a locus that gives as a result the activation of said sequence. Another method implies modification of the nucleic acid sequence, or its control sequences, before introducing it into the embryo. Another method consists of transfecting the embryo using a vector that contains the nucleic acid sequence to be introduced.

In a particular embodiment, the introduction of the DNA construct of the invention in the germ line of a non-human mammal is performed by means of microinjection of a linear DNA fragment that comprises the activatable gene operatively bound to the promoter that directs the expression in Sca-1⁺ cells in fertilized oocytes of non-human mammals.

The fertilised oocytes can be isolated by conventional methods, for example, provoking the ovulation of the female, either in response to copulation with a male or by induction by treatment with the luteinising hormone. In general, a superovulation is induced in the females by hormonal action and they are crossed with males. After an appropriate period of time, the females are sacrificed to isolate the fertilised oocytes from their oviducts, which are kept in an appropriate culture medium. The fertilised oocytes can be recognised under the microscope by the presence of pronuclei. The microinjection of the linear DNA fragment is performed, advantageously, in the male pronucleus.

After the introduction of the linear DNA fragment that comprises the DNA construct of the invention in fertilised oocytes, they are incubated *in vitro* for an appropriate period of time or else they are reimplanted in pseudopregnant wet nursing mothers (obtained by making female copulate with sterile males). The implantation is performed by conventional methods, for example, anaesthetising the females and surgically inserting a sufficient number of embryos, for example, 10-20 embryos, in the oviducts of the pseudopregnant wet nursing mothers. Once gestation is over, some embryos will conclude the gestation and give rise to non-human transgenic mammals, which theoretically should carry the DNA construct of the invention integrated into their genome and present in all the cells of the organism. This progeny is the G0 generation and their individuals are the "transgenic founders". The confirmation that an individual has incorporated the injected nuclear acid and is transgenic is obtained by analysing the individuals of the progeny. To do this, from a sample of animal material, for example, from a small sample from the animal's tail (in the event that it is, for example, a mouse) or a blood example, the DNA is extracted from each individual and analysed by conventional methods, for example, by polymerase chain reaction (PCR) using the specific initiators or by Southern blot or Northern blot analysis using, for example, a probe that is complementary to, at least, a part of the transgene, or else by Western blot analysis using an antibody to the protein coded by the transgene. Other methods for evaluating the presence of the transgene include, without limitation, appropriate biochemical assays, such as enzymatic and/or immunological assays, histological staining for particular markers, enzymatic activities, etc.

In general, in transgenic animals, the inserted transgene is transmitted as a Mendelian characteristic and so it is not difficult to establish the stable lines of each individual. If the G0 individuals are crossed with the parent strain (retrocrossing) and the transgene behaves with Mendelian characteristics, 50% of the progeny will be heterozygotic for the inserted transgene (hemizygotic). These individuals constitute the G1 progeny and a transgenic line that can be maintained indefinitely, crossing hemizygotics of the G1 generation with normal individuals. Alternatively, individuals of the G1 generation can be crossed among themselves to produce 25% homozygotics for the inserted transgene, 50% hemizygotics and 25% without the transgene provided the transgene does not affect the viability of the descendents.

The progeny of a non-human transgenic mammal provided by this invention, such as the progeny of a transgenic mouse provided by this invention can be obtained, therefore, by copulation of the transgenic animal with an appropriate individual, or by *in vitro* fertilization of eggs and/or sperm of the transgenic animals. As used in this description, the term "progeny" or "progeny of a non-human transgenic mammal" relates to all descendents of a previous generation of the non-human transgenic mammals originally transformed. The progeny can be analysed to detect the presence of the transgene by any of the aforementioned methods.

The invention also relates to a non-human transgenic mammal cell line that contains a DNA construct of the invention on its genome. In a particular embodiment, said cell line is a murine cell line.

The transgenic non-human mammal provided by this invention, its progeny or the cell line provided by this invention, are useful for, among other applications, evaluating potentially useful compounds for treating and/or preventing a genetic anomaly associated with neoplastic or non-neoplastic human pathology of stem cell origin or of non stem cell origin. Therefore, the invention also refers to the use of said non-human transgenic mammal, its progeny or a cell line provided by this invention, in the evaluation of potentially useful compounds for the treatment and/or prevention of a genetic anomaly associated with a human pathology. In a particular embodiment, said human pathology is a human pathology of stem cell origin.

In the case of transgenic animals, the evaluation of the potentially useful compound for the treatment and/or prevention of said human pathology of stem cell origin can be performed by administration of the compound to be tested to said transgenic animal, at different doses, and evaluating the physiological response of the animal over time. The administration of the compound to be assayed can be oral or parenteral, depending on the chemical nature of the compound to be evaluated. In some cases, it may be appropriate to administer the compound in question along with cofactors that enhance the effect of the compound.

In the case of cell lines of the invention, the evaluation of the potentially useful compound for the treatment and/or prevention of said human pathology of stem cell origin can be performed by adding the compound to be assayed to a cell culture medium for an appropriate period of time, at different concentrations, and evaluating the cellular response to the compound over time using appropriate biochemical and/or histological assays. At times, it may be necessary to add the compound in question to the cellular culture medium along with cofactors that enhance the effect of the compound.

The following examples illustrate the invention and should not be considered limiting the scope thereof.

### EXAMPLE 1

### Murine CSC after treatment with ganciclovir (GCV)

### I. MATERIALS AND METHODS

### Production of transgenic PLy6-HSVtk-IRES-BCRABLp210 mice

For the generation of the transgenic non-human mammal provided by this invention, the DNA construct of the invention, HSVtk-IRES-BCRABLp210, has been introduced into said mammal, or into a predecessor thereof, in an embryonic state, for example, in the state of a cell, or fertilized oocyte and, generally, not later than the g cell state.

The procedure for the preparation of a transgenic non-human mammal that possesses this chromosomal anomaly associated with a human pathology of stem cell origin, is as follows:
(i) introducing a DNA construct of the invention into a fertilized oocyte of a non-human transgenic mammal;
(ii) implanting said fertilized oocyte into a pseudopregnant wet nursing mother to produce descendants; and
(iii) analysing said descendants to evaluate the existence of activated genes and/or genes created by a chromosomal anomaly associated with a human pathology of stem cell origin.

### Ganciclovir (GVC) treatment

PLy6-HSVtk-IRES-BCRABLp210 mice wcrc used to test the effectiveness of GCV-induced cell in CSC that express BCR-ABL. GCV, after preliminary testing, was administered at a dose of 100 mg/kg/day by i.p. injection for 14 days. This dose has been reported to kill cells expressing HSV-tk in transgenic mice (Bush TG. Cell 1998, 93: 189-201). Dosing started when the mice were leukaemic. A control group was given injections of normal saline.

### RNA extraction

Total RNA was isolated in two steps using TRIzol (Life Technologies, Inc., Grand Island, NY) followed by Rneasy Mini-Kit (Quiagen Inc., Valencia, CA) purification following the manufacturer's RNA Clean-up protocol with the optional On-column Dnase treatment. The integrity and the quality of RNA were verified by electrophoresis and its concentration was measured.

### II. RESULTS

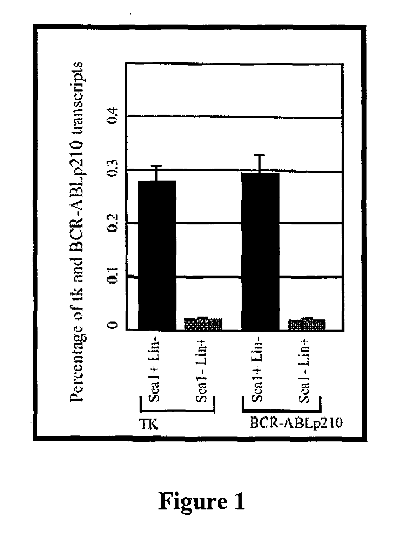

Analysis of transgene expression, thymidine kisase and BCR-ABLp210, in Sca1⁺Lin⁻ and Scal⁻Lin⁺ lines was studied. Results showed high level of expression of both transgenes only in the Scal⁺Lin⁻ lines (see figure 1). This expression pattern confirmed a selective transgene expression in the cancer stem cells (Sca1⁺Lin⁻).

In Table 1 the different cancer types developed in PLy6-HSVtk-IRES-BCRABLp210 mice are represented. The results demonstrate that said transgenic mice developed the same tumour pattern as the one observed in humans affected with the BCRABLp210 genetic anomaly.

**Table 1**

| **Cancer development pattern in PLy6-HSVtk-IRES-BCRABLp210 mice** | |
|---|---|
| CML only | 81% |
| CML + Hepatic ADC | 3% |
| CML + Fibrohistiocytoma | 2% |
| CML + Sarcoma osteogenic | 2% |
| CML + Tumor cell. Sertoli | 2% |
| CML + Lung ADC | 10% |

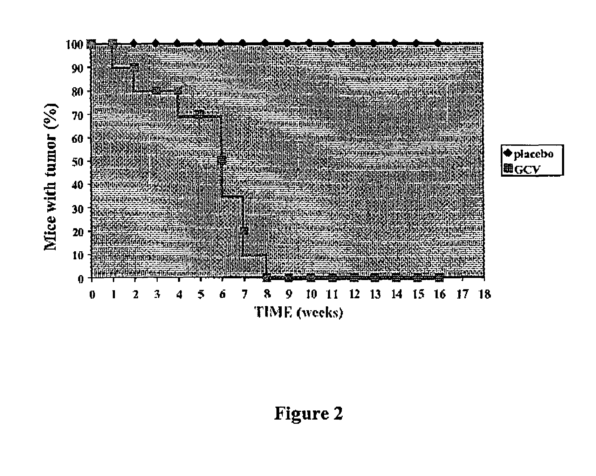

30 mice were used as a control and were subjected daily with saline solution whereas the 60 remaining mice were injected daily with GCV as described in method section. During this period, cancer disappearing completely and these mice, carrying the cancers that disappeared following treatment, were observed for an additional 3 months in which no tumor recurrence was observed (CML and carcinomas) (Fig. 2).

### EXAMPLE 2

### Genetic profile of murine CSC

### I. MATERIALS AND METHODS

### RNA Extraction

Total RNA was isolated in two steps using TRIzol (Life Technologies, Inc., Grand Island, NY) followed by Rneasy Mini-Kit (Qiagen Inc., Valencia, CA) purification following the manufacturer's RNA Clean-up protocol with the optional On-column Dnase treatment. The integrity and the quality of RNA were verified by electrophoresis and its concentration measured.

### Target Preparation

T-7-based RNA amplifications and preparations of cDNA probes were performed as described previously (Van Gelder RN, von Zastrow ME, Yool A, Dement WC, Barchas JD, Ebcrwine JH: Amplified RNA synthesized from limited quantities of heterogeneous cDNA. Proc Natl Acad Sci USA 1990, 87:1663-1667. Eberwine J: Amplification of mRNA populations using aRNA generated from immobilized oligo(dT)-T7 primed cDNA. Biotechniques 1996, 20:584-591). Briefly, a maximum amount of 5 µg of total RNA were converted to double-stranded cDNA using the superscript choice system (Life Technologies) using oligo-dT primer containing a T7 RNA polymerase promoter. The double-stranded cDNA was cleaned up, and T7 *in vitro* transcription was performed using Megascript T7 *in vitro* transcription kit (Ambion, Austin, TX) following the manufacturers' instructions.

### Microarray Procedure

2.5 µg of second round amplified RNA from each sample was directly labeled with cyanine 3-conjugated dUTP (Cy3), whereas 2.5 □g of second round amplified RNA from the Universal Mouse Reference RNA (Stratagene) was labeled with cyanine 5-conjugated dUTP (Cy5) as reference. For all microarray studies the CNIO MouseChip was used. Hybridizations were performed as described. After washing, the slides were scanned using a Scanarray 5000 XL (GSI Lumonics Kanata, Ontario, Canada) and images were analyzed with the GenePix 4.0 program (Axon Instruments Inc., Union City, CA).

### Data Analysis

Data obtained from each hybridization were stored in a database for analysis. The Cy3:Cy5 ratios were normalized to the median ratio value of all of the spots in the array. After normalization, spots with intensities for both channels (sum of medians) less than that of the local background were discarded. The ratios of the remaining spots were log transformed (base 2), and duplicated spots on the MouseChip were averaged to the median. To obtain the expression profile of cancer stem cells (CSC), we referred the ratios of the CSC to the control hematopoietic stem cells. Genes were selected to be upregulated or down-regulated if the difference in ratio was at least 2-fold. For clustering análisis, the SOTA clustering program (Yuspa SH et al. Cancer Res 1980; 40:4694-703) was used, and trees were viewed using the TreeView program.

### II. RESULTS

Microarrays results showed a different expression profile of cancer stem cell before and afterwards lymphoma development. These data show significant differences in three markers: Bcl-2, mdm2 and GATA-3. Bcl-2 marker presented a significant decrease in gene expression after lymphoma development compared to control levels. On the other hand, the last two markers, mdm2 and GATA-3, showed an increased response after lymphoma development compared to control (see Table 2).

**Table 2**

| **CSC (Sca1**^{**+**}**/Lin**^{**-**}**)** | | |
|---|---|---|
| | **Lymphoma development** | |
| | **Before** | **After** |
| **Bmi-1** | + | |
| **Nanog** | - | |
| **PU.1** | + | + |
| **Pax-5** | + | +/- |
| **E2A** | - | |
| **GATA3** | - | + |
| **c-Kit** | - | |
| **CEBPg/z** | + | + |
| **R-IL3** | - | |
| **Bcl2** | + | - |
| **Bid** | + | + |
| **Bak** | + | +/- |
| **PCDC2** | + | +/- |
| **p21** | + | |
| **p53** | - | |
| **mdm2** | - | + |
| **P27** | | |
| **CCND2** | - | |

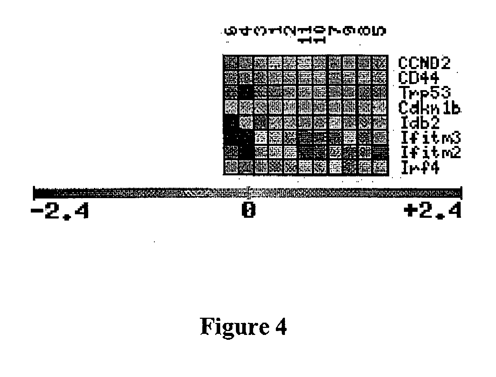

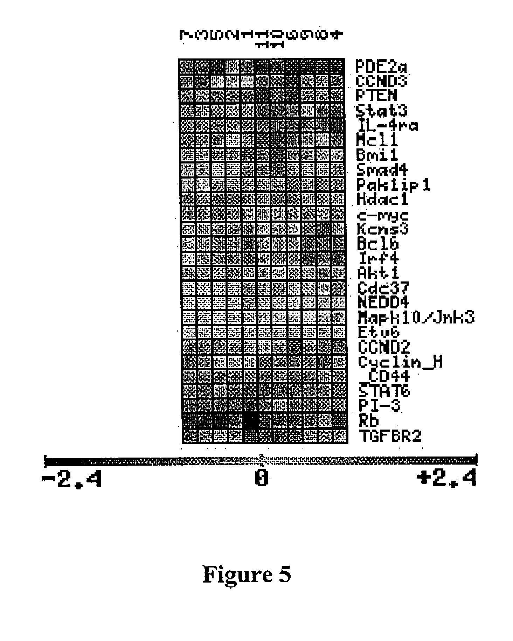

In figure 4 it is shown a gene expression pattern corresponding to cancer stem cells from Bcl6 mice (Sca1⁺ Lin⁻). On the order hand, the murine cancer stem cells harboring BCL6 transgene show a similar expression profile (Figure 5) to the lymphoma human B-cells (Alizadeh AA, Eisen MB, Davis RE, Ma C, Lossos IS, Rosenwald A, Boldrick JC, Sabet H, Tran T, Yu X, Powell JI, Yang L, Marti GE, Moore T, Hudson J Jr, Lu L, Lewis DB, Tibshirani R, Sherlock G, Chan WC, Greiner TC, Weisenburger DD, Armitage JO, Warnke R, Levy R, Wilson W, Grever MR, Byrd JC, Botstein D, Brown PO, Staudt LM. 2000. Distinct types of diffuse large B-cell lymphoma identified by gene expression profiling. Nature.Feb 3;403(6769): 503-11). It shows the murine cancer stem cells harboring the Bcl6 transgene can be used as model to mimic human lymphoma.

## Claims

1. A murine stem cell comprising a gene created and/or activated by a genetic anomaly associated with a human pathology.

2. Stem cell according to claim 1, wherein said stem cell is a cancer stem cell.

3. Stem cell according to claim 1, wherein said human pathology is a human pathology of stem cell origin.

4. Stem cell according to claim 1, wherein said human pathology is a human epithelial or mesenchimal cancer.

5. Stem cell according to claim 4, wherein said human pathology is selected from lymphomas, leukaemias, sarcomas and carcinomas.

6. Stem cell according to claim 5, wherein said human pathology is selected from chronic mycloid leukaemia, B-cell acute lymphoblastic leukaemia, T-cell acute lymphoblastic leukaemia, acute myeloid leukaemia, chronic myeloid leukaemia, lymphoproliferative syndromes, multiple myeloma, liposarcoma, Ewing sarcoma, lung carcinoma, breast carcinoma, skin carcinoma and kidney carcinoma.

7. Stem cell according to claim 1, further comprising a marker.

8. Stem cell according to claim 7, wherein said marker is a marker useful for the specific isolation and/or identification of cancer stem cells, or for the procurement of cancer stem cells, or for the differentiation of cancer stem cells from health stem cells.

9. Stem cell according to claim 1, further comprising a reporter.

10. Stem cell according to claim 9, wherein said reporter is a reporter useful for spatiotemporal identification of the onset, progression, dissemination and further physiopathological processes, for evaluating the effect of therapies by molecular imaging techniques, for diagnostic assays, drug discovery and development processes, for target identification and for improving the efficacy and reliability of all phases of the clinical development.

11. Use of a murine stem cell according to anyone of claims 1 to 10 as a biomarker for:
- detecting the presence of a gene created and/or activated by a genetic anomaly associated with a human pathology in a subject, or for
- assessing the risk or predisposition of a subject to develop a human pathology in a subject, or for
- determining the stage or severity of a human pathology in a subject, or for
- monitoring the effect of the therapy administered to a subject having a human pathology, or for
- designing an individualized therapy for a subject suffering from a human pathology, or for
- designing human clinical trials, or for
- diagnosis of cancer and/or specific processes and effects of cancer development, like cancer dissemination; or for
- patient selection for personalized therapeutics; or for
- therapeutic monitoring and evaluation of therapeutic benefits; or for
- drug discovery and pharmacokinetics guidance.

12. Use of a murine stem cell according to anyone of claims 1 to 10, for discovering, screening, searching, identifying, evaluating and validating targets for human pathologies, or for identifying specific genes related to self-renewal ability of cancer stem cells.

13. A method for detecting the presence of a gene created and/or activated by a genetic anomaly associated with a human pathology in a subject or for assessing the risk or predisposition of a subject to develop said pathology which comprises identifying a stem cell in a sample from said subject, said stem cell comprising a genetic anomaly associated with said human pathology of stem cell origin.

14. A method for determining the stage or severity of a human pathology in a subject or for monitoring the effect of the therapy administered to a subject having said pathology, which comprises identifying and quantifying the concentration of stem cells in a sample from said subject, said stem cells comprising a gene created and/or activated by a genetic anomaly associated with a human pathology, and comparing said concentration with that of a control sample or with a prior sample from said subject or with a prior sample from said subject taken before administering a therapy.

15. Method according to claim 13 or 14, wherein said stem cell is a cancer stem cell.

16. Method according to anyone of claims 13 to 15, wherein said human pathology is a human pathology of stem cell origin.

17. A method for screening, searching, identifying, discovering, developing and/or evaluating compounds for treating a human pathology or for repositioning known drugs or combinations of compounds, which comprises contacting a candidate compound with a murine stem cell according to anyone of claims 1 to 10 and monitoring the response.

18. A method for designing an individualized therapy for a human suffering from a human pathology which comprises selecting a compound identified according to claim 17, wherein said compound abolishes or slows down said cancer stem cells proliferation or kills said stem cells, said compound being used as active principle in a pharmaceutical composition to be administered to said subject.

19. A method for designing human clinical trials which comprises:
(i) selecting targets for murine stem cells according to anyone of claims 1 to 10 or molecular profiling said murine stem cells;
(ii) validating and, optionally, optimizing, said target or said molecular profile in a disease state; and
(iii)selecting responders vs non-responders.

20. A DNA construct comprising a gene created and/or activated by a genetic anomaly associated with a human pathology operatively bound to a promoter that directs the expression of said genetic anomaly in Sca-1⁺ cells, wherein said genetic anomaly is selected from (i) a nucleic acid comprising a BCL6 gene and (ii) a nucleic acid comprising a first nucleotide sequence coding for a kinase and a second nucleotide sequence comprising BCR-ABL^{p210}.

21. DNA construct according to claim 20, wherein said gene created and/or activated by a genetic anomaly comprises a nucleic acid comprising a first nucleotide sequence coding for herpex simple thymidine kinase (HSV-tk), a second nucleotide sequence comprising BCR-ABL^{p210}, and a third nucleotide sequence comprising an internal ribosome-entry site (IRES) sequence, wherein 3' end of said first nucleotide sequence is bound to the 5' end of said third nucleotide sequence, and the 3' end of said third nucleotide sequence is bound to the 5' end of said second nucleotide sequence.

22. DNA construct according to anyone of claims 20 or 21, wherein said promoter that directs the expression of said gene created and/or activated by a genetic anomaly in Sca-1⁺ cells is the mouse promoter pLy-6E.1 or a functional fragment thereof.

23. A transgenic non-human mammal that contains in its genome a DNA construct according to the anyone of claims 20 to 22.

24. Transgenic non-human mammal according to claim 23, wherein said mammal is a rodent, preferably, a mouse or a rat.

25. The progeny of a transgenic non-human mammal according to anyone of claims 23 or 24.

26. A cell line of a transgenic non-human mammal according to anyone of claims 23 or 24, said cell line containing in its genome a DNA construct according to anyone of claims 20 to 22.

27. Cell line according to claim 26, wherein said cell line is a murine cell line.

28. A process for the preparation of a transgenic non-human mammal that possesses a genetic anomaly associated with a human pathology of stem cell origin, which comprises:
a) introducing a DNA construct according to anyone of claims 20 to 23 into a fertilised oocyte of a non-human transgenic mammal;
b) implanting said fertilised oocyte in a pseudopregnant wet nursing mother to produce descendents; and
c) analysing said descendants to evaluate the existence of a genetic anomaly associated with a human pathology of stem cell origin.

29. Process according to claim 28, wherein said non-human mammal is a rodent, preferably a mouse or a rat.
